# EUROPEAN PATENT APPLICATION

(11) **EP 4 371 588 A1**
(43) Date of publication of application: **22.05.2024**
(21) Application number: 22841735.8
(22) Date of filing: 25.03.2022
(51) Int. Cl.: A61L 101/10, A61L 101/22, A61L 2/20, F24F 11/46, F24F 11/64, F24F 11/72, F24F 11/79, F24F 110/20, F24F 120/10, F24F 6/00, F24F 8/24

(54) **METHOD FOR SUPPRESSING VIRUS ACTION AND DEVICE FOR OUTPUTTING VIRUS ACTION SUPPRESSING COMPONENT**

(30) Priority: 14.07.2021 JP 2021116478
(71) Applicant: Sharp Kabushiki Kaisha, Sakai-shi Osaka 590-8522 (JP)
(72) Inventor: OKAJIMA, Hiromasa, Sakai-ku, Sakai City Osaka 590-8522 (JP); KUBO, Yukihiro, Sakai-ku, Sakai City Osaka 590-8522 (JP); UENISHI, Akira, Sakai-ku, Sakai City Osaka 590-8522 (JP); YAMAMOTO, Satohiko, Sakai-ku, Sakai City Osaka 590-8522 (JP)
(74) Representative: Treeby, Philip David William
(86) International application number: PCT/JP2022/014579
(87) International publication number: WO 2023/286385

(57) **Abstract**

An apparatus for delivering a virus action suppressing component performs, when delivering an active ingredient including a virus action suppressing component into a space, detecting a humidity in the space, determining whether the humidity in the space is equal to or higher than a first set value, and increasing an amount of the active ingredient delivered into the space when the humidity in the space is equal to or higher than the first set value, compared to when the humidity in the space is less than the first set value. This can increase the effect of suppressing the action of viruses when the humidity is high.

## Description

### Technical Field

The present invention relates to a method for suppressing virus action and an apparatus for delivering a virus action suppressing component.

### Background Art

It is known in the prior art that, for example, the risk of infection by influenza viruses is lower when the humidity is high than when the humidity is low. It is also known that droplets generated, for example, by people coughing or sneezing which are one of the causes of infection by viruses (including bacteria), are more likely to be scattered when the humidity is low than when the humidity is high. This is because the drier the air, that is, the lower the humidity, the easier it is for droplets to evaporate and become aerosols (become fine particles and float around in a space). Conversely, the higher the humidity, the more moisture is contained in the air, which makes it more difficult for droplets to evaporate, thus suppressing their scattering. However, while scattering is suppressed, the amount of droplets that fall and adhere to floors or the like tend to increase.

An air purifier with a humidifying function (hereinafter referred to as an air purifier), which can increase the humidity in a space and deliver a component for suppressing the action of viruses to lower the risk of infection by viruses (including bacteria) and suppress the scattering of droplets, is known in the prior art. A general air purifier increases the humidity in a space in which the air purifier is installed using a humidifying device to suppress the scattering of droplets and protect people's throats from drying out. The air purifier also delivers a component for suppressing the action of viruses (hereinafter referred to as an active ingredient) into the space to suppress the action of viruses.

An air purifier described in PTL 1 includes a blower device, a humidifying device, and a component generating device (a negative ion generating device) for suppressing the action of viruses. In the air purifier described in PTL 1, an air flow delivered from the blower device first passes through the humidifying device, then passes through the negative ion generating device, and finally flows into the space. Then, the air purifier can perform humidifying or deliver air containing negative ions into the space to humidify the space or suppress the action of viruses present in the space. Generally, when the humidity reaches a set value (generally, a humidity of 55% to 60%) by humidifying the space, such a conventional air purifier prevents the humidity in the space from increasing any further, and in order to stop humidifying, reduces the air flow volume of a blower device provided in the air purifier or stops the blower device. Then, when the humidity in the space drops to a certain level from the set value, the air purifier increases the air flow volume of the blower device if it is low and starts the blower device if it is stopped to humidify the space again to the set value and maintain the humidity at the set value. This is because maintaining the humidity at the set value can continuously protect people's throats and suppress the action of viruses. Also with respect to SARS-CoV-2 (hereinafter referred to as coronavirus), which has been prevalent in recent years, such air purifiers are expected to be effective in suppressing the action of viruses.

### Citation List

### Patent Literature

PTL 1: JP 4069915 B

### Summary of Invention

### Technical Problem

However, according to experiments using coronaviruses conducted by the applicant (details will be described later), it was found that the infectivity (the proportion of infectious viruses) of coronaviruses contained in saliva adhering to floors, walls, or the like which are a concern for human infection is higher when the humidity is high than when the humidity is low. Here, as described above, prior art air purifiers generally use a humidifying device to humidify the space, and if the humidity in the space reaches a set value (e.g., a humidity of 60%), prevent the humidity in the space from increasing any further, and in order to stop humidifying, perform control to reduce the air flow volume of a blower device provided in the air purifier or stop the blower device. In this case, reducing the air flow volume of the blower device or stopping the blower device will also reduce the amount of the active ingredient delivered into the space from a virus action suppressing component-generating device that is present in the same path as the blower device and the humidifying device.

According to experiments that will be described later, the infectivity of coronaviruses contained in saliva adhering to a floor, walls, and the like that define a space is higher when the humidity is high than when the humidity is low and thus it is undesirable from the perspective of the risk of infection to humans or the like that, as with prior art air purifiers, the amount of the active ingredient delivered from the air purifier into the space decreases when the humidity is high.

Therefore, it is an object of the present invention to provide a method for suppressing virus action and an apparatus for delivering a virus action suppressing component which can increase the effect of suppressing the action of viruses even when the humidity is high.

### Solution to Problem

The most important feature of the present invention includes, when delivering an active ingredient including a virus action suppressing component into a space, detecting a humidity in the space, determining whether the humidity in the space is equal to or higher than a first set value, and increasing an amount of the active ingredient delivered into the space when the humidity in the space is equal to or higher than the first set value, compared to when the humidity in the space is less than the first set value.

### Advantageous Effects of Invention

According to the above solution, the amount of the active ingredient including the virus action suppressing component delivered into the space is increased when the humidity becomes high. Thus, even when the humidity is high, it is possible to sufficiently suppress the action of viruses, compared to the case where, when the humidity reaches a set value (becomes high), the humidity in the space is prevented from increasing any further as with prior art air purifiers and control is performed to decrease the air flow volume of the blower device or stop the blower device in order to stop humidifying, such that the amount of the active ingredient delivered into the space is reduced due to the presence of a virus action suppressing component-generating device in the same path as the blower device and the humidifying device.

### Brief Description of Drawings

FIG. 1 is a bar graph showing the results of a first experiment regarding changes in infectivity of coronaviruses.
FIG. 2 is a bar graph showing the results of a second experiment regarding changes in infectivity of coronaviruses.
FIG. 3 is a block diagram illustrating an apparatus for delivering a virus action suppressing component.
FIG. 4 is flowchart (1) illustrating an embodiment.
FIG. 5 is flowchart (2) illustrating the embodiment.
FIG. 6 is flowchart (3) illustrating the embodiment.
FIG. 7 is flowchart (4) illustrating the embodiment.
FIG. 8 is a side view illustrating an apparatus for delivering a virus action suppressing component when the humidity is less than a first set value.
FIG. 9 is a side view illustrating the apparatus for delivering a virus action suppressing component when the humidity is equal to or higher than the first set value.

### Description of Embodiments

### Embodiments

### Description of Experimental Results

Experiments conducted by the applicant will be described in detail below.

It is known that in the case of influenza virus, for example, the risk of virus infection is lower when the humidity is high than when the humidity is low as described above. It is also known that there is a tendency that the higher the humidity, the more scattering of droplets generated, for example, by people coughing or sneezing which are a concern for infection by viruses or the like, into the space will be suppressed, but the amount of droplets that fall to floors or the like will increase accordingly.

Thus, the applicant conducted two experiments to investigate whether coronaviruses, which have been prevalent worldwide in recent years, have the same tendency of infection risk as influenza viruses and investigate the tendency of the infectivity (the proportion of infectious viruses) of coronaviruses contained in droplets (saliva) generated, for example, by people coughing or sneezing adhering to floors or the like which are a concern for infection.

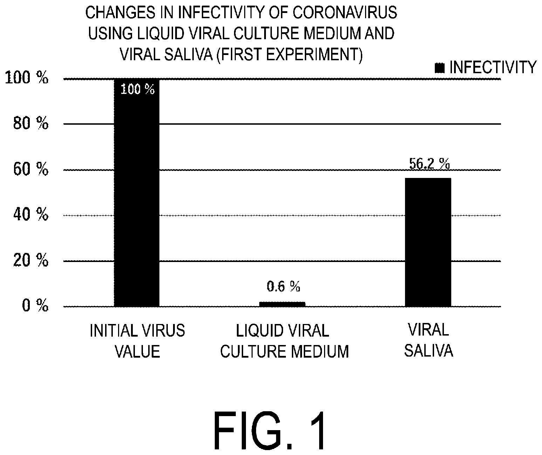

FIG. 1 is a bar graph showing the results of a first experiment.

In the first experiment, an experiment was conducted to evaluate the infectivity of viruses after two hours in a 60% humidity environment using a liquid culture medium containing coronaviruses (a liquid viral culture medium), which is generally used in experiments for evaluating infectivity, and human saliva containing coronaviruses (viral saliva). Here, experimental conditions were that (1) room temperature was 23°C, (2) saliva from 7 adults regardless of gender was used, (3) a culture medium D-MEM/Ham's F-12 was used as a liquid culture medium, (4) 50 µL of a liquid viral culture medium and 50 µL of viral saliva was applied to an experimental filter, and (5) the method of evaluating infectivity was a 50% tissue culture infectious dose (TCID 50 method).

When the infectivity of the liquid viral culture medium and the viral saliva was evaluated after two hours, the infectivity of the liquid viral culture medium was reduced to 0.6% of the initial value of 100%, while the infectivity of the viral saliva was reduced only to 56.2%.

For coronaviruses, this experiment showed that in a 60% humidity environment, the infectivity of viruses contained in saliva is significantly higher than that of viruses contained in a culture medium commonly used in experiments. Incidentally, when the infectivity of the liquid viral culture medium was evaluated after two hours in a 30% humidity environment, the infectivity was reduced to 0.1%.

From this, it was found that the infectivity of the liquid viral culture medium was reduced significantly after two hours in both a 60% humidity environment and a 30% humidity environment.

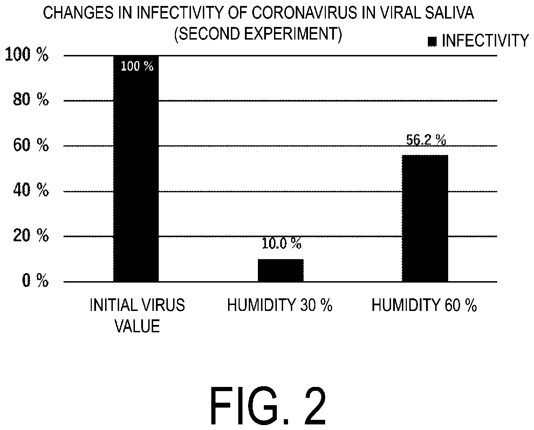

FIG. 2 is a bar graph showing the results of a second experiment.

In the second experiment, an experiment was conducted to investigate whether coronaviruses contained in saliva adhering to floors or the like which were shown to have high infectivity in the first experiment were affected by the reduction in infectivity depending on the humidity level. Specifically, in addition to the first experiment described above, an experiment was also conducted using viral saliva to evaluate the infectivity of viruses after two hours when the humidity was 30%. The experimental conditions were the same as in the first experiment.

When the infectivity of the viral saliva was evaluated after two hours, the infectivity was reduced to 10% of the initial value of 100% when the humidity was 30%.

That is, the applicant has newly discovered that the infectivity of coronaviruses contained in saliva is higher when the humidity is high than when the humidity is low.

Prior art air purifiers generally use a humidifying device to humidify a space, and if the humidity reaches a set value (generally, a humidity of 55% to 60%), prevent the humidity in the space from increasing any further, and in order to stop humidifying, perform control to reduce the air flow volume of a blower device provided in the air purifier or stop the blower device. In this case, reducing the air flow volume of the blower device or stopping the blower device will also reduce the amount of the active ingredient delivered into the space from the virus action suppressing component-generating device that is present in the same path as the blower device and the humidifying device.

According to the applicant's findings based on the applicant's experiments, the reduction in the amount of the active ingredient delivered into the space is undesirable from the perspective of infection risk or the like because the infectivity of coronaviruses contained in saliva is higher when the humidity is high than when the humidity is low.

Therefore, the present invention increases the amount of the virus action suppressing component (the active ingredient) delivered into the space when the humidity becomes high. A method and apparatus for suppressing the action of viruses according to the present invention will be described in detail below.

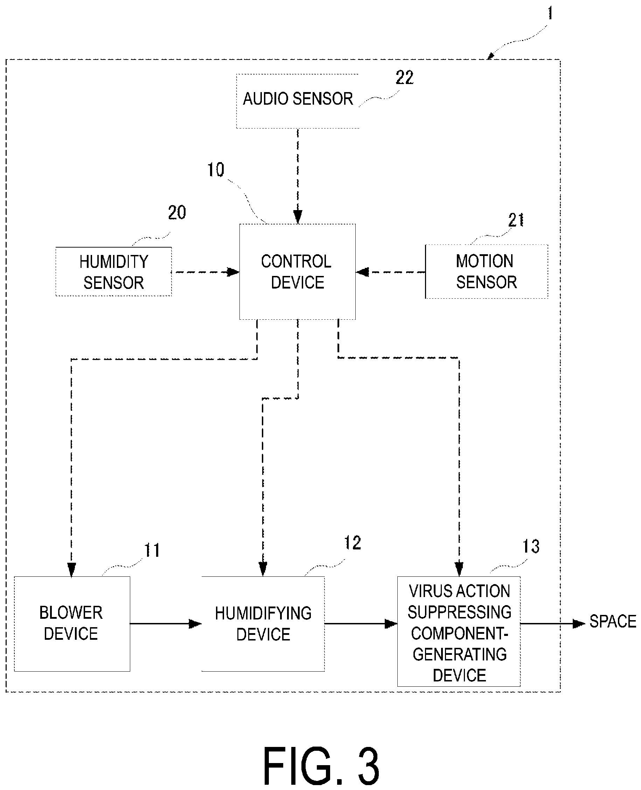

FIG. 3 is a block diagram illustrating an apparatus 1 that delivers a virus action suppressing component, that is, an active ingredient, into a space (hereinafter referred to as a component delivery apparatus 1). In FIG. 3, arrows in solid lines indicate flows of air delivered from a blower device 11 and arrows in broken lines indicate flows of signals.

The component delivery apparatus 1 includes the above-mentioned blower device 11 for delivering an air flow into the space, a humidifying device 12 for humidifying the space, and a component generating device 13 for generating a virus action suppressing component, that is, an active ingredient. The component delivery apparatus 1 also includes a control device 10 for controlling the blower device 11, the humidifying device 12, and the component generating device 13. The component delivery apparatus 1 further includes a humidity sensor 20, a motion sensor 21, and an audio sensor 22 that send control signals to the control device 10.

The blower device 11 includes, for example, a propeller fan and a motor. The blower device 11 is provided to deliver an air flow out of the component delivery apparatus 1. The air flow generated by the blower device 11 is delivered from the blower device 11 to the humidifying device 12, humidified in the humidifying device 12, flows from the humidifying device 12 to the component generating device 13, where the air flow is supplied with an active ingredient and then finally delivered into the space. The air flow delivered into the space can increase the humidity in the space and suppress the action of viruses. The blower device 11 is controlled such that it weakens or strengthens the air flow volume or starts or stops based on a signal from the control device 10.

The humidifying device 12 is, for example, of an evaporative type that includes a filter containing moisture for humidification. An air flow delivered from the blower device 11 passes through the moisture-containing filter of the humidifying device 12 and the air flow that has passed through the moisture-containing filter is delivered into the space, thereby making it possible to humidify the space. The humidifying device 12 is controlled to start or stop its humidifying function based on a signal from the control device 10.

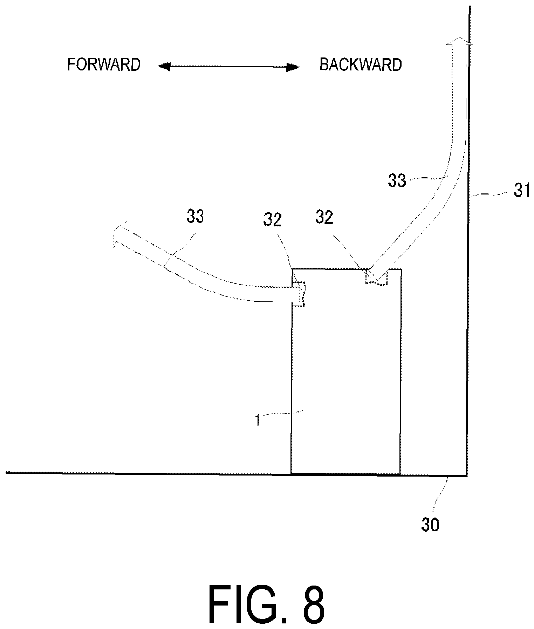

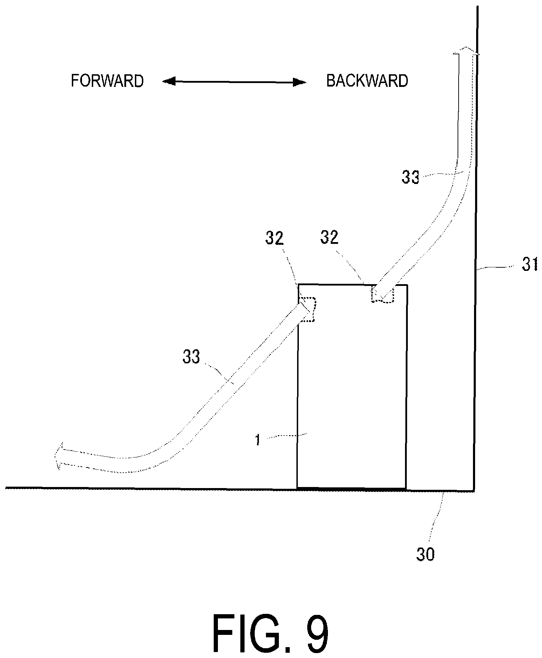

The component generating device 13 is provided near an air outlet into the space (see an air outlet 32 in FIG. 8 and FIG. 9) of the component delivery apparatus 1 in order to deliver a component including a virus action suppressing component, that is, an active ingredient, into the space. The active ingredient generated by the component generating device 13 is delivered into the space by the air flow delivered from the blower device 11. Examples of the active ingredient generated by the component generating device 13 include ions or active species. The ions include positive ions (e.g., H⁺(H₂O)ₘ (where m is any integer)), negative ions (e.g., O₂⁻(H₂O)ₙ (where n is any integer)), or a combination thereof. Examples of the active species include hydroxy radicals (•OH), hydrogen radicals (•H), oxygen radicals (•O), hydroperoxy radicals (•HO₂), hydrogen peroxide (H₂O₂), and ozone (O₃).

The component generating device 13 is capable of increasing or decreasing the amount of an active ingredient delivered based on a signal from the control device 10. More specifically, for example, the component generating device 13 is capable of controlling the delivery amount by increasing or decreasing the delivery amount of the active ingredient itself or is capable of controlling the delivery amount by increasing or decreasing the concentration of the active ingredient.

The control device 10 includes, for example, a central processing unit (CPU). The control device 10 is capable of receiving signals from the humidity sensor 20, the motion sensor 21, and the audio sensor 22 and is capable of transmitting signals to the blower device 11, the humidifying device 12, and the component generating device 13 to control the same.

The humidity sensor 20 is provided, for example, on an outer back surface of the component delivery apparatus 1 in order to detect the humidity in the space in which the component delivery apparatus 1 is provided. Upon detecting the humidity in the space, the humidity sensor 20 transmits a signal to the control device 10, such that a humidity detection signal is transmitted to the control device 10 and the control device 10 determines the humidity.

The motion sensor 21 is provided, for example, on an outer front surface of a case of the component delivery apparatus 1 in order to detect human presence. The motion sensor 21 detects whether there is a person in the space, and upon detecting human presence, sends a signal to the control device 10. A signal detecting human presence is transmitted to the control device 10, and the control device 10 determines whether there is a person in the space.

The audio sensor 22 is provided, for example, on the outer back surface of the case of the component delivery apparatus 1 in order to detect the intensity of a human voice. Upon detecting the intensity of a human voice in the space, the audio sensor 22 transmits a signal to the control device 10 and the control device 10 determines the intensity of the human voice. The audio sensor 22 is not necessarily configured to selectively detect the intensity of a human voice and may be configured to detect the intensity of sound including a human voice. Similarly, the control device 10 is not necessarily configured to selectively determine the intensity of a human voice and may be configured to determine the intensity of sound including a human voice. This is because when a sound is heard in a space, it is highly likely that there is a person in that space.

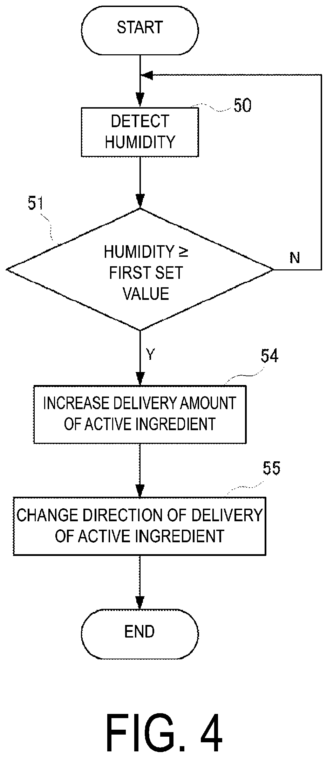

A processing method according to the present embodiment will be described below based on FIGS. 4 to 7.

FIG. 4 is a flowchart illustrating the embodiment. The control device 10 performs operations illustrated in the flowchart of FIG. 4 at fixed intervals. When the processing illustrated in FIG. 4 starts, the processing first proceeds to step 50 in which the humidity sensor 20 detects the humidity in the space. Next, a humidity detection signal is sent from the humidity sensor 20 to the control device 10 and the processing proceeds to step 51 in which the control device 10 determines whether the detected humidity in the space is equal to or higher than a first set value. Here, the first set value is an arbitrary value. In the present embodiment, the first set value is a humidity of 60%. When the control device 10 has determined that the detected humidity in the space is equal to or higher than the first set value (60%), the processing proceeds to the step 54 of increasing the amount of the active ingredient delivered into the space compared to when the humidity in the space is less than the first set value (60%).

In step 54, the control device 10 sends a signal to either or both of the blower device 11 and the component generating device 13 to increase the amount of the active ingredient delivered into the space. In a method of increasing the amount of the active ingredient, the control device 10 performs control to increase the amount of the active ingredient delivered into the space by sending a signal to the blower device 11 to increase the air flow volume. Alternatively, the control device 10 performs control to increase the amount of the active ingredient delivered into the space by sending a signal to increase the delivery amount of the active ingredient itself generated by the component generating device 13 or by sending a signal to increase the concentration of the active ingredient.

After the step 54 of increasing the amount of the active ingredient delivered into the space, the processing proceeds to step 55 in which the control device 10 changes the direction of delivery of the active ingredient into the space. The control device 10 can also end the series of processing after step 54 without performing the processing of step 55. Although details will be described later, the control device 10 changes the delivery direction such that the active ingredient is delivered in a direction toward a wall surface and a direction toward a floor surface that define the space. Specifically, the delivery direction is changed such that the active ingredient is delivered backward and upward and forward and downward from the component delivery apparatus 1. The delivery direction of the active ingredient is changed and the active ingredient is delivered in a direction toward the floor surface (forward and downward from the component delivery apparatus 1) and in a direction toward the wall surface (backward and upward from the component delivery apparatus 1), thereby actively suppressing the action of viruses adhering to the floor, the wall, and the like.

If the detected humidity in the space is less than the first set value (60%) in step 51, the processing returns to before step 50 in which the humidity sensor 20 detects the humidity in the space and then proceeds again to step 50 in which the humidity sensor 20 detects the humidity in the space.

By increasing the amount of the active ingredient delivered into the space when the humidity is high, it is possible to suppress the action of viruses with high infectivity contained in saliva in a high humidity environment.

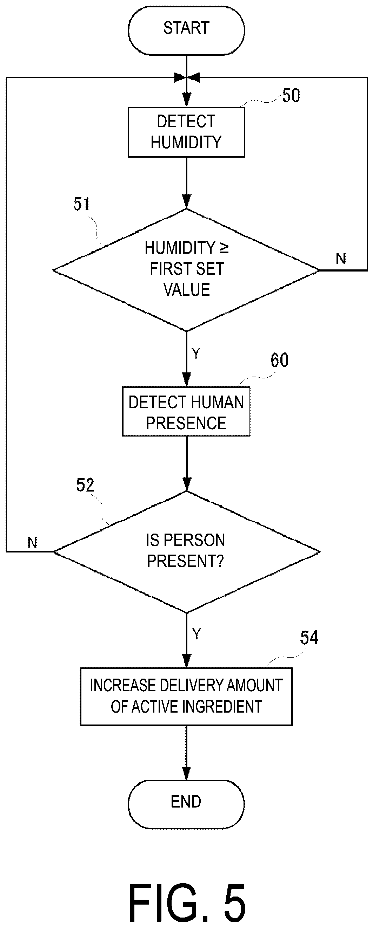

FIG. 5 is a flowchart illustrating the embodiment. The control device 10 performs operations illustrated in the flowchart of FIG. 5 at fixed intervals. When the processing illustrated in FIG. 5 starts, the processing first proceeds to step 50 in which the humidity sensor 20 detects the humidity in the space. Next, a humidity detection signal is sent from the humidity sensor 20 to the control device 10 and the processing proceeds to step 51 in which the control device 10 determines whether the detected humidity in the space is equal to or higher than a first set value (60%). If the detected humidity in the space is equal to or higher than the first set value (60%), the processing proceeds to step 60 in which the motion sensor 21 performs human presence detection in the space. When the motion sensor 21 has detected human presence, a detection signal is sent from the motion sensor 21 to the control device 10 and the processing proceeds to step 52 in which the control device 10 determines whether there is a person in the space. When the control device 10 has determined that there is a person in the space, the processing proceeds to the step 54 of increasing the amount of the active ingredient delivered into the space compared to when the humidity in the space is less than the first set value (60%) and human presence is not detected.

In step 54, the control device 10 sends a signal to either or both of the blower device 11 and the component generating device 13 to increase the amount of the active ingredient delivered into the space. In a method of increasing the amount of the active ingredient, the control device 10 performs control to increase the amount of the active ingredient delivered into the space by sending a signal to the blower device 11 to increase the air flow volume. Alternatively, the control device 10 performs control to increase the amount of the active ingredient delivered into the space by sending a signal to increase the delivery amount of the active ingredient itself generated by the component generating device 13 or by sending a signal to increase the concentration of the active ingredient.

If the detected humidity in the space is less than the first set value (60%) in step 51 or if human presence is not detected in step 52, the processing returns to before step 50 in which the humidity sensor 20 detects the humidity in the space and then proceeds again to step 50 in which the humidity sensor 20 detects the humidity in the space.

If there are people in a space, droplets may fall on the floor or the like when they talk, cough, or sneeze. When the humidity in the space is high, more droplets fall on the floor or the like than when the humidity is low, and there is a risk that people will come into contact with the fallen droplets, increasing infection risk. Additionally, if a person comes from outside, there is a possibility that viruses or the like have adhered to the person's clothes or the like. Thus, if a person is detected in a space and the humidity in the space is equal to or higher than the first set value (60%), it is possible to suppress the action of viruses with high infectivity or the like contained in saliva in a high humidity environment by increasing the amount of the active ingredient delivered into the space.

According to the idea described above, it is also effective to perform control to increase the amount of the active ingredient delivered into the space when the motion sensor 21 has detected that there is a person in the space while the humidity in the space is less than the first set value (60%).

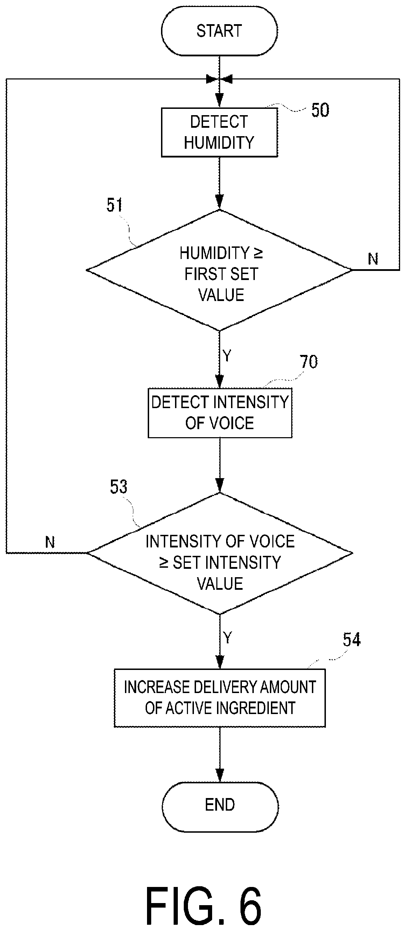

FIG. 6 is a flowchart illustrating the embodiment. The control device 10 performs operations illustrated in the flowchart of FIG. 6 at fixed intervals. When the processing illustrated in FIG. 6 starts, the processing first proceeds to step 50 in which the humidity sensor 20 detects the humidity in the space. Next, a humidity detection signal is sent from the humidity sensor 20 to the control device 10 and the processing proceeds to step 51 in which the control device 10 determines whether the detected humidity in the space is equal to or higher than a first set value (60%). If the detected humidity in the space is equal to or higher than the first set value (60%), the processing proceeds to step 70 in which the audio sensor 22 detects the intensity of a human voice. When the intensity of a human voice is detected, a detection signal is sent from the audio sensor 22 to the control device 10 and the processing proceeds to step 53 in which the control device 10 determines whether the detected intensity of the human voice is equal to or higher than a set intensity value. Here, the set intensity value is an arbitrary value. In the present embodiment, the set intensity value is 60 dB. When the intensity of the human voice is equal to or higher than the set intensity value (60 dB), the processing proceeds to the step 54 of increasing the amount of the active ingredient delivered into the space compared to when the humidity in the space is less than the first set value (60%) and the intensity of the human voice is less than the set intensity value (60 dB).

In step 54, the control device 10 sends a signal to either or both of the blower device 11 and the component generating device 13 to increase the amount of the active ingredient delivered into the space. In a method of increasing the amount of the active ingredient, the control device 10 performs control to increase the amount of the active ingredient delivered into the space by sending a signal to the blower device 11 to increase the air flow volume. Alternatively, the control device 10 performs control to increase the amount of the active ingredient delivered into the space by sending a signal to increase the delivery amount of the active ingredient itself generated by the component generating device 13 or by sending a signal to increase the concentration of the active ingredient.

If the detected humidity in the space is less than the first set value in step 51 or if the intensity of the human voice is less than the set intensity value (60 dB) in step 53, the processing returns to before step 50 in which the humidity sensor 20 detects the humidity in the space and then proceeds again to step 50 in which the humidity sensor 20 detects the humidity in the space.

By increasing the amount of the active ingredient delivered into the space when the humidity is high, i.e., when a lot of droplets generated by people talking fall, it is possible to suppress the action of viruses or the like contained in saliva which have high infectivity (in a high humidity environment).

According to the same idea as described above, in this case as well, it is effective to perform control to increase the amount of the active ingredient delivered into the space when the intensity of the human voice is equal to or higher than the set intensity value (60 dB) while the humidity in the space is less than the first set value (60%).

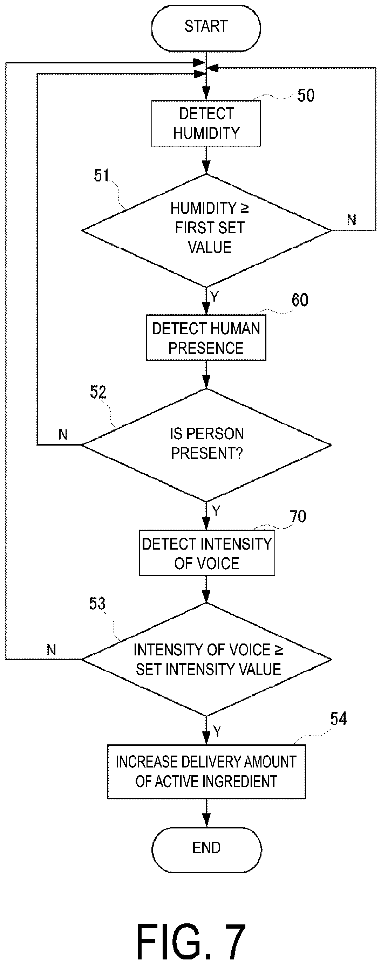

FIG. 7 is a flowchart illustrating the embodiment. The control device 10 performs operations illustrated in the flowchart of FIG. 7 at fixed intervals. When the processing illustrated in FIG. 7 starts, the processing first proceeds to step 50 in which the humidity sensor 20 detects the humidity in the space. Next, a humidity detection signal is sent from the humidity sensor 20 to the control device 10 and the processing proceeds to step 51 in which the control device 10 determines whether the detected humidity in the space is equal to or higher than a first set value (60%). If the detected humidity in the space is equal to or higher than the first set value (60%), the processing proceeds to step 60 in which the motion sensor 21 performs human presence detection in the space.

When the motion sensor 21 has detected human presence, a detection signal is sent from the motion sensor 21 to the control device 10 and the processing proceeds to step 52 in which the control device 10 determines whether there is a person in the space. When the control device 10 has determined that there is a person in the space, the processing proceeds to step 70 in which the audio sensor 22 detects the intensity of a human voice. When the intensity of a human voice is detected, a detection signal is sent from the audio sensor to the control device 10 and the processing proceeds to step 53 in which the control device 10 determines whether the detected intensity of the human voice is equal to or higher than a set intensity value (60 dB). When the intensity of the human voice is equal to or higher than the set intensity value (60 dB), the processing proceeds to the step 54 of increasing the amount of the active ingredient delivered into the space compared to when the humidity in the space is less than the first set value (60%), human presence is not detected, and the intensity of the human voice is less than the set intensity value (60 dB).

In step 54, the control device 10 sends a signal to either or both of the blower device 11 and the component generating device 13 to increase the amount of the active ingredient delivered into the space. In a method of increasing the amount of the active ingredient, the control device 10 performs control to increase the amount of the active ingredient delivered into the space by sending a signal to the blower device 11 to increase the air flow volume. Alternatively, the control device 10 performs control to increase the amount of the active ingredient delivered into the space by sending a signal to increase the delivery amount of the active ingredient itself generated by the component generating device 13 or by sending a signal to increase the concentration of the active ingredient.

If the detected humidity in the space is less than the first set value in step 51, if human presence is not detected in step 52, or if the intensity of the human voice is less than the set intensity value in step 53, then the processing returns to before step 50 in which the humidity sensor 20 detects the humidity in the space and then proceeds again to step 50 in which the humidity sensor 20 detects the humidity in the space.

When the humidity in the space is equal to or higher than the first set value (60%), the motion sensor 21 has detected human presence, and the intensity of the human voice detected by the audio sensor 22 is equal to or higher than the set intensity value (60 dB), this means that the humidity is high and a lot of droplets generated by people talking fall. By increasing the amount of the active ingredient delivered into the space in such a case, it is possible to suppress the action of viruses or the like contained in saliva which have high infectivity (in a high humidity environment).

Although not illustrated in FIGS. 4 to 7, it is also preferable to perform the step of humidifying the space using the humidifying device 12. In this case, if the control device 10 determines that the humidity detected by the humidity sensor 20 is equal to or higher than the second set value, a signal is sent from the control device 10 to the humidifying device 12 such that the humidifying device 12 stops functioning. Here, the second set value is an arbitrary value and may be the same as or different from the first set value. In the present embodiment, the second set value is set to a humidity of 60%, which is the same as the first set value. When the space is humidified by the humidifying device 12 and the humidity in the space rises to the second set value of humidity (60%), it is possible to prevent the humidity in the space from reaching the second set value (60%) or higher by stopping the function of the humidifying device 12. When the humidity is equal to or higher than the second set value (60%), it is also effective to, after stopping the function of the humidifying device 12, maintain or increase the air flow volume delivered into the space from the blower device 11 compared to when the humidity is less than the second set value (60%) to prevent the amount of the active ingredient delivered into the space from being reduced compared to when the humidity is less than the second set value (60%). By doing so, it is possible to suppress the action of viruses or the like contained in saliva which have high infectivity (in a high humidity environment).

In a method of increasing the amount of the active ingredient delivered to the space, the control device 10 may perform control to increase the amount of the active ingredient delivered into the space by sending a signal to the component generating device 13 to increase the amount of the active ingredient itself generated by the component generating device 13 without changing the air flow volume of the blower device 11 from when the humidity is less than the first set value (60%). Alternatively, control may be performed to increase the amount of the active ingredient delivered into the space by sending a signal to increase the concentration of the active ingredient generated by the component generating device 13 without changing the air flow volume of the blower device 11 from when the humidity is less than the first set value (60%).

In a different method of increasing the amount of the active ingredient delivered to the space, the control device 10 may perform control to increase the amount of the active ingredient delivered into the space by sending a signal to increase the air flow volume of the blower device 11 without changing the delivery amount of the active ingredient itself generated by the component generating device 13 and the concentration of the active ingredient from when the humidity is less than the first set value (60%).

It is also effective for the control device 10 to perform control to maintain the air flow volume of the blower device 11 and the delivery amount of the active ingredient itself generated by the component generating device 13 or the concentration of the active ingredient at the same air flow volume and the same delivery amount or the same concentration as when the humidity is less than the first set value (60%).

With such a configuration, it is possible to increase the amount of the active ingredient delivered into the space when the humidity reaches the first set value (60%) or higher. Thus, it is possible, for example, to sufficiently suppress the action of viruses or the like which are at a high humidity, i.e., which have high infectivity, compared to the case where, when the humidity reaches a set value (becomes high), the humidity in the space is prevented from increasing any further as with prior art air purifiers or the like and control is performed to decrease the air flow volume of the blower device 11 or stop the blower device 11 in order to stop humidifying, thus reducing the delivery amount of the active ingredient from the component generating device 13 which is present in the same path as the blower device 11 and the humidifying device 12.

FIG. 8 and FIG. 9 are side views illustrating directions in which the component delivery apparatus 1 delivers air flows. The component delivery apparatus 1 is installed on a floor 30 in a space near a wall 31 with its back facing the wall. The component delivery apparatus 1 has air outlets 32 on its front and top surfaces and delivers air flows 33 through the air outlets 32. When the humidity is less than the first set value (60%), the directions of air flows 33 delivered from the component delivery apparatus 1 are arbitrary, but for example, as illustrated in FIG. 8, an air flow 33 is delivered toward the wall 31 located above and behind the component delivery apparatus 1 through the air outlet 32 located on the top surface of the component delivery apparatus 1, thereby circulating air in the space along the wall 31 and a ceiling (not illustrated) that define the space. Through the air outlet 32 on the front surface of the component delivery apparatus 1, an air flow 33 containing an active ingredient is delivered toward the space located above and in front of the component delivery apparatus 1 in order to suppress the action of viruses or the like floating in the space.

The humidity sensor 20 sends the humidity to the control device 10, and when the control device 10 has determined that the humidity in the space is equal to or higher than the first set value (60%), the delivery direction in which an air flow 33 is delivered through the air outlet 32 of the component delivery apparatus 1 is either or both of a direction toward the floor 30 located below and in front of the component delivery apparatus 1 and a direction toward the wall 31 located above and behind the component delivery apparatus 1. In FIG. 9, air flows 33 are delivered in both of the direction toward the floor 30 and the direction toward the wall 31. The delivery direction is changed, for example, by louvers (not illustrated) provided at the air outlets 32. The operations of the louvers (not illustrated) are controlled by the control device 10.

Applying the active ingredient delivered from the component generating device 13 to viruses or the like adhering to the floor 30 located below and in front of and the wall 31 located above and behind the component delivery apparatus 1 can suppress the action of viruses or the like contained in saliva which have high infectivity (in a high humidity environment). An air outlet 32 may be provided only on the front surface or only on the top surface of the component delivery apparatus 1. An air flow 33 delivered through the air outlet 32 provided on the front surface of the component delivery apparatus 1 can suppress the action of viruses or the like adhering to the floor 30. An air flow 33 delivered through the air outlet 32 provided on the top surface of the component delivery apparatus 1 can suppress the action of viruses or the like adhering to the wall 31.

The present technique is not limited to increasing the air flow volume of the blower device 11 or increasing the concentration of the active ingredient generated by the component generating device 13 and can use any other method as long as it can increase the amount of the active ingredient delivered into the space. For example, a component may be ejected directly into the space through a nozzle without using air flow power or a component generating device 13 with a function to adjust the amount of generation may be installed near an opening formed in the case of the component delivery apparatus 1 and the same component may be delivered into the space directly from the component generating device 13.

In a method of increasing the air flow power of the blower device 11, for example, either or both of the air flow volume and the air flow rate may be increased by increasing the rotation speed of the propeller fan (not illustrated) of the blower device 11 or the air flow rate may be increased by reducing the opening areas of the air outlets 32 provided in the component delivery apparatus 1 using the louvers (not illustrated) or the like.

Ions and active species that are active ingredients generated by the component generating device 13 are effective not only for suppressing the action of viruses as described above but also for microorganism elimination (including sterilization).

Embodiments of the present invention have been described above with reference to the drawings. However, the present invention is not limited to the above embodiments and can be embodied in various modes without departing from the spirit of the present invention. To facilitate understanding, the drawings schematically illustrate each component emphasized, and the thickness and length of each component illustrated, the number of components illustrated, the spacing therebetween, and the like differ from the actual ones for convenience of drawing. The material, shape, dimensions, and the like of each component shown in the above embodiment are examples and are not particularly limited and various modifications are possible without substantially departing from the configuration of the present invention.

### Industrial Applicability

The apparatus for delivering a virus action suppressing component according to the present invention can sufficiently suppress the action of viruses even when the humidity in the space is high and can also be effective against coronaviruses that have been prevalent in recent years, such that it has industrial applicability and its value is fully recognized.

## Claims

1. A method for suppressing virus action, the method comprising:
when delivering an active ingredient including a virus action suppressing component into a space, detecting a humidity in the space;
determining whether the humidity in the space is equal to or higher than a first set value; and
increasing an amount of the active ingredient delivered into the space when the humidity in the space is equal to or higher than the first set value, compared to when the humidity in the space is less than the first set value.

2. The method for suppressing virus action according to claim 1,
wherein the increasing of the amount of the active ingredient delivered into the space includes increasing air flow power for delivering the active ingredient into the space by the air flow power.

3. The method for suppressing virus action according to claim 1 or 2, the method further comprising:
humidifying the space; and
stopping the humidifying when the humidity in the space is equal to or higher than a second set value.

4. The method for suppressing virus action according to any one of claims 1 to 3, the method further comprising:
detecting human presence in the space; and
increasing the amount of the active ingredient delivered into the space when the humidity in the space is equal to or higher than the first set value and human presence in the space is detected, compared to when the humidity in the space is less than the first set value and human presence in the space is not detected.

5. The method for suppressing virus action according to any one of claims 1 to 4, the method further comprising:
detecting an intensity of a human voice in the space; and
increasing the amount of the active ingredient delivered into the space when the humidity in the space is equal to or higher than the first set value and the intensity of the human voice in the space is equal to or higher than a set intensity value, compared to when the humidity in the space is less than the first set value and the intensity of the human voice in the space is less than the set intensity value.

6. The method for suppressing virus action according to any one of claims 1 to 5, the method further comprising
setting a delivery direction of the active ingredient to either or both of a direction toward a wall surface and a direction toward a floor surface, the wall surface and the floor surface defining the space, when the humidity in the space is equal to or higher than the first set value.

7. The method for suppressing virus action according to claim 6,
wherein the setting of the delivery direction of the active ingredient to either or both of the direction toward the wall surface and the direction toward the floor surface, the wall surface and the floor surface defining the space, when the humidity in the space is equal to or higher than the first set value includes setting, by using an apparatus for delivering a virus action suppressing component, the delivery direction to either or both of a direction backward and upward and a direction forward and downward from the apparatus, the apparatus being configured to deliver the active ingredient.

8. An apparatus for delivering a virus action suppressing component, the apparatus comprising:
a virus action suppressing component-generating device configured to deliver an active ingredient including a virus action suppressing component into a space;
a humidity sensor configured to detect a humidity in the space; and
a control device configured to perform control to determine whether the humidity in the space is equal to or higher than a first set value and increase an amount of the active ingredient delivered into the space when the humidity in the space is equal to or higher than the first set value, compared to when the humidity in the space is less than the first set value.

9. The apparatus for delivering a virus action suppressing component according to claim 8, the apparatus further comprising
a blower device configured to generate an air flow and deliver the active ingredient into the space by the air flow,
wherein the control device performs control to increase the amount of the active ingredient delivered into the space by increasing an air flow volume of the blower device when the humidity in the space is equal to or higher than the first set value.

10. The apparatus for delivering a virus action suppressing component according to claim 8 or 9, the apparatus further comprising
a humidifying device configured to humidify the space,
wherein the humidifying device stops the humidifying when the humidity in the space is equal to or higher than a second set value.

11. The apparatus for delivering a virus action suppressing component according to any one of claims 8 to 10, the apparatus further comprising
a motion sensor configured to detect human presence in the space,
wherein the control device performs control to increase the amount of the active ingredient delivered into the space when the humidity in the space is equal to or higher than the first set value and the motion sensor has detected the human presence in the space, compared to when the humidity in the space is less than the first set value and the motion sensor has not detected the human presence in the space.

12. The apparatus for delivering a virus action suppressing component according to any one of claims 8 to 11, the apparatus further comprising
an audio sensor configured to detect an intensity of a human voice in the space,
wherein the control device performs control to increase the amount of the active ingredient delivered into the space when the humidity in the space is equal to or higher than the first set value and the intensity of the human voice in the space detected by the audio sensor is equal to or higher than a set intensity value, compared to when the humidity in the space is less than the first set value and the intensity of the human voice in the space is less than the set intensity value.

13. The apparatus for delivering a virus action suppressing component according to any one of claims 8 to 12,
wherein the apparatus is configured to set a delivery direction of the active ingredient to either or both of a direction toward a wall surface and a direction toward a floor surface, the wall surface and the floor surface defining the space, when the humidity in the space is equal to or higher than the first set value.

14. The apparatus for delivering a virus action suppressing component according to claim 13,
wherein the apparatus delivers the active ingredient in either or both of a direction backward and upward from the apparatus, the direction backward and upward being the direction toward the wall surface, and a direction forward and downward from the apparatus, the direction forward and downward being the direction toward the floor surface.
